# EUROPEAN PATENT SPECIFICATION

(11) **EP 2 349 123 B1**
(45) Date of publication and mention of the grant of the patent: **04.11.2015**
(21) Application number: 09740926.2
(22) Date of filing: 12.10.2009
(51) Int. Cl.: A61F 2/958

(54) **A MEDICAL DEVICE**
MEDIZINISCHE VORRICHTUNG
DISPOSITIF MÉDICAL

(30) Priority: 10.10.2008 US 249448; 10.10.2008 EP 08253309
(43) Date of publication of application: 03.08.2011
(73) Proprietor: Veryan Medical Limited, Oxford Business Park Oxford, OX4 2HN (GB)
(72) Inventor: HERATY, Kevin, Co. Galway (IE); MULLINS, Liam, Galway (IE); GILSON, Paul, County Galway (IE); BURKE, Martin, Sussex RH13 6LW (GB); BLOWICK, Ray, Galway (IE)
(74) Representative: Piésold, Alexander James
(86) International application number: PCT/GB2009/002433
(87) International publication number: WO 2010/041038

(56) References cited:
- EP-A- 0 714 640
- EP-A- 1 279 382
- WO-A-02/066095
- WO-A1-2004/082533
- WO-A2-2008/117256
- GB-A- 2 425 485
- US-A1- 2001 049 549
- US-A1- 2007 112 407
- US-A1- 2007 213 663

## Description

This invention relates to a medical device.

US 2001/049549 discloses a stent having a curve shaped to match the curve of the vessel in which it is to be deployed. The stent may be expanded by a balloon.

WO 2008/117256 discloses a helical balloon catheter that adopts a helical formation upon inflation.

WO2004/082533 relates to a helical stent, it also teaches that a balloon for expanding a stent may be formed in a helical shape.

EP 1279382 also relates to a stent having a curvature adapted to match the curvature of an implantation site within a patient's body lumen. It discloses a medical device comprising: a balloon for location in a blood vessel, the balloon having at each end an end part; the balloon being inflatable between a collapsed configuration and an expanded configuration; wherein in the expanded configuration at least part of the longitudinal axis of the balloon is curved in three-dimensional space.

The present invention is characterised in that in the expanded configuration at least part of the balloon is substantially helically shaped; and wherein a longitudinal axis of each end part of the balloon has a helical angle that varies along the length of the end part and which increases in a direction away from the end of the balloon.

In one embodiment of the invention in the expanded configuration the balloon (expandable element) is configured to exert force on the internal wall of a blood vessel causing the longitudinal axis of the blood vessel to curve in three-dimensional space. Blood flowing through the three-dimensional curved part of the blood vessel undergoes a swirling action. The swirling flow of blood has been found to minimise thrombosis and platelet adhesion, and to minimise or prevent coverage of a stent by ingrowth of intima. The flow pattern in the blood vessel including the swirling pattern induced by the non-planar geometry of the blood vessel operates to inhibit the development of vascular diseases such as thrombosis/atherosclerosis and intimal hyperplasia. Preferably the device comprises a single expandable element. However the device may alternatively comprise a plurality of separate expandable members.

In one case in the collapsed configuration at least part of the longitudinal axis of the expandable element is substantially straight. This arrangement provides a low-profile for ease of delivery. Preferably in the collapsed configuration at least part of the expandable element is substantially cylindrically shaped. In the expanded configuration at least part of the expandable element may be substantially helically shaped. In the expanded configuration at least part of the expandable element may be substantially spiral shaped.

Each longitudinal end part of the balloon (expandable element) has a helical angle which varies along the length of the end part. The helical angle of the longitudinal axis of an end part of the expandable element increases in a direction away from the end of the element. When a blood vessel is caused by such an expandable element to have a helical longitudinal axis, the end part of the expandable element causes the helical angle of the longitudinal axis of the blood vessel to increase along the length of the end part. Thus a transitional region may be provided in the vessel in which flow in the part of the vessel shaped by the end part of the expandable element is conditioned by the varying helical angle. This arrangement can serve to reduce the area of the internal wall of the blood vessel which has low wall shear stress, to reduce the possibility of recirculation, and to reduce the risk of neointimal hyperplasia. Wall shear stress is generated on the internal wall of a blood vessel by flow adjacent to the wall. Higher levels of wall shear stress have been associated with a reduction in levels of in-stent restenosis.

The expandable element may be configured to at least partially expand a stent. Preferably in the expanded configuration the expandable element is configured to exert force via a stent on the internal wall of a blood vessel. The expandable element may be configured to expand a stent having a longitudinal axis which curves in three-dimensional space. The expandable element may be configured to expand a stent having a longitudinal axis which is substantially straight. Ideally the device comprises a stent deployment device.

Alternatively, the stent with a longitudinal axis which curves in three-dimensional space may have been deployed at a previous time and the expandable element is configured to exert force on the stent to recreate or reinforce this shape, should the shape have become less defined over time, or if the three dimensional shape is not adequate from the outset (i.e. if shape only partially taken up by vessel on implantation of the stent).

Most preferably the device comprises means to align the expandable element with a stent or blood vessel lumen. In the case of a three-dimensional curved stent it is particularly important to ensure the stent is correctly aligned with the three-dimensional curve of the expandable element. The alignment means may comprise means to visualise the expandable element. The alignment means may be arranged to assist rotational alignment of the expandable element with the stent. The alignment means may be arranged to assist axial alignment of the expandable element with the stent. Preferably the alignment means is arranged to assist rotational and axial alignment. Preferably the alignment means comprises one or more markers on the expandable element.

The markers may comprise radiopaque material such as tantalum, platinum, gold or iridium. The markers may be located on diametrically opposite sides of the expandable element, such that when an x-ray source is applied to the expandable element inserted in the stent, the x-ray image produced can be used to determine the orientation of the expandable element. For example, an image showing both markers may indicate the expandable element is out of alignment, whilst an image showing only one marker (the other being in the shadow of the first) indicates the expandable element is aligned.

In another embodiment, the alignment means extends circumferentially and/or longitudinally along at least part of the expandable element, for example in a cross or partial cross shape.

Preferably, the stent is also provided with markers, which are located so as to be positioned in a predetermined way relative to the markers on the stent when the expandable element is correctly aligned within the stent. For example, the stent can be provided with radiopaque markers which line up with those on the expandable element when the latter is correctly aligned.

In another embodiment markers are provided on both the expandable element and the stent such that when the expandable element is inserted inside the collapsed stent, the markers can be used to achieve correct alignment. For example the markers on the expandable element and the stent may match up. The markers may also be used to achieve correct alignment between the expandable element and the stent when the stent has been inserted in the body and the expandable element is expanded within the stent.

However, in some cases, due to the small size of the delivery system involved in transluminal procedures, it might be difficult to distinguish individual markers on devices. For example, it might be difficult to view the rotational orientation of an asymmetric marker such as an "L" on an expandable element. With this in mind, an embodiment of the invention provides another alternative alignment means. In this embodiment, the expandable element comprises a varying cross section. When inflated with saline or a contrast enhancing medium (radiopaque dye), the varying cross-section of the expandable element will be visible when viewed using fluoroscopy (the use of a radiopaque dye in particular to fill the expandable element aids its visualisation during inflation and positioning). The varying cross section can be used to determine the rotational orientation of the expandable element and thus enable it to be deployed correctly. The expandable element may be deployed and aligned in a blood vessel or stent within a blood vessel in-vivo, or deployed and aligned in a stent ex-vivo prior to insertion in the body.

Using the capability of the c-arm on a fluoroscope, for example, the expandable element can be viewed in two orthogonal directions, for example in elevation (lateral) and plan (anterior posterior) view. In this way, the orientation of the varying or notched cross-section of the expandable element can be determined whilst it is inserted into a vessel or stent within the body, and thus the expandable element can be orientated correctly with respect to the curvature of the stent or vessel lumen.

The cross section may vary in any way that enables the orientation of the expandable element to be identified. For example, the variation may be such that a projection view of the cross section of the expandable element varies as the expandable element is rotated. In one embodiment the variation in cross section preferably comprises a notch, which may be any shape or indentation in the outer surface of the expandable element. Such a notch preferably has first order rotational symmetry. Preferably a notch comprises a substantially flat portion recessed in the outer surface of the expandable element. One such notch is preferably provided towards each end of the expandable element.

In one embodiment, a stent is provided with at least one reference point, for example a radiopaque marker, which is arranged to line up with the varying cross section (or a portion thereof) of the expandable element in a pre-determined manner when the expandable element is correctly deployed. In one embodiment, radiopaque markers are positioned so as to match with the notched portions of the expandable element when the expandable element is rotated and deployed correctly. The markers can be any shape, but are preferably elliptical or rectangular. Suitable radiopaque materials are tantalum, platinum, gold or iridium.

The variation in cross section, for example a notched portion, preferably aligns uniquely in only one way with a marker on a stent. Thus, once alignment with the marker is achieved, rotational alignment with the stent is also achieved.

In one embodiment, two radiopaque markers are provided, one towards each end of the stent. However the markers are more preferably provided in pairs, each pair comprising two markers arranged diametrically opposite, i.e. 180° apart, around the circumference of the stent. In another embodiment multiple pairs are provided at the same longitudinal position on the stent, each pair being arranged diametrically opposite and being distinguishable from other pairs. For example two pairs of markers may be provided, each of the four markers being separated by 90°.

Thus, as can be seen, the above described alignment means ensures that a certain rotational orientation of the expandable element with respect to the stent or vessel geometry is achieved when deployed.

Whilst this alignment means is particularly useful to assist the positioning of an expandable element having a longitudinal axis curved in three-dimensional space, in accordance with present invention, it is also useful for aligning other expandable elements.

A method for treating a blood vessel may comprise the steps of: locating an expandable element in the blood vessel; moving the expandable element from a collapsed configuration to an expanded configuration; and aligning the expandable element within the blood vessel or a stent located within the blood vessel using a portion of the expandable element having a varying or notched cross section. This is therefore an in-vivo procedure, carried out inside the body.

A method of manufacturing a medical device may comprise: locating an expandable element in a stent; and aligning the expandable element within the stent using markers on or a varying cross section of the expandable element. Preferably markers on the stent are also used to align the expandable element. This is an ex-vivo procedure, carried out outside the body.

Preferably, the step of aligning comprises aligning the varying or notched cross section portion with a corresponding reference point on the stent.

Preferably, in the expanded configuration of the expandable element, the expandable element is configured to exert force, via a stent in which it is inserted, on the internal wall of a blood vessel to deform the internal wall of the blood vessel such that the longitudinal axis of the blood vessel is caused to curve in three-dimensional space.

In one embodiment in the expanded configuration the expandable element is configured to contact the internal wall of a blood vessel directly, i.e. no stent is used. Preferably the device comprises a dilation device.

In one case the device comprises an elongate element upon which the expandable element is mounted.

The device of the invention may be employed as a stent delivery device, and/or as a stent deployment device, and/or as a post dilatation device.

In another aspect of the invention there is provided a medical system comprising:-
a medical device of the invention, and
a stent suitable for deployment in a blood vessel to support at least part of the internal wall of the blood vessel.

In one embodiment of the invention the stent is movable between a delivery configuration and a deployed configuration. Preferably the stent is collapsed in the delivery configuration. This low-profile arrangement provides for ease of delivery. Ideally in the delivery configuration at least part of the longitudinal axis of the stent is substantially straight. Most preferably the stent is expanded in the deployed configuration. In the deployed configuration at least part of the longitudinal axis of the stent may be curved in three-dimensional space. Preferably in the deployed configuration the stent is configured to exert force on the internal wall of a blood vessel causing the longitudinal axis of the blood vessel to curve in three-dimensional space. Blood flowing through the three-dimensional curved part of the blood vessel undergoes a swirling action. The swirling flow of blood has been found to minimise thrombosis and platelet adhesion, and to minimise or prevent coverage of the stent by ingrowth of intima. The flow pattern in the blood vessel including the swirling pattern induced by the non-planar geometry of the blood vessel operates to inhibit the development of vascular diseases such as thrombosis/atherosclerosis and intimal hyperplasia.

In one case the stent is movable between the delivery configuration and an intermediate configuration, and between the intermediate configuration and the deployed configuration. Preferably the stent is at least partially expanded in the intermediate configuration. In the intermediate configuration at least part of the longitudinal axis of the stent may be substantially straight. In the intermediate configuration at least part of the longitudinal axis of the stent may be curved in three-dimensional space.

A method for treating a blood vessel may comprise the steps of:-
locating an expandable element in the blood vessel, and
moving the expandable element from a collapsed configuration to an expanded configuration in which at least part of the longitudinal axis of the expandable element is curved in three-dimensional space.

In one embodiment of the invention the expandable element exerts force on the internal wall of the blood vessel causing the longitudinal axis of the blood vessel to curve in three-dimensional space. Blood flowing through the three-dimensional curved part of the blood vessel undergoes a swirling action. The swirling flow of blood has been found to minimise thrombosis and platelet adhesion, and to minimise or prevent coverage of a stent by ingrowth of intima. The flow pattern in the blood vessel including the swirling pattern induced by the non-planar geometry of the blood vessel operates to inhibit the development of vascular diseases such as thrombosis/atherosclerosis and intimal hyperplasia.

In one case the step of moving the expandable element from the collapsed configuration to the expanded configuration at least partially expands a stent. Preferably the expandable element exerts force via the stent on the internal wall of the blood vessel. Ideally the method comprises the step of moving the stent from a delivery configuration to a deployed configuration. Most preferably the method comprises the step of moving the stent from the delivery configuration to an intermediate configuration, and from the intermediate configuration to the deployed configuration. The invention may provide a method of deploying a stent. Preferably the method comprises the step of aligning the expandable element with a stent. In the case of a three-dimensional curved stent it is important to ensure the stent is correctly aligned with the three-dimensional curve of the expandable element.

In another embodiment the expandable element contacts the internal wall of the blood vessel directly. The invention may provide a method of dilation.

The invention will be more clearly understood from the following description of some embodiments thereof, given by way of example only, with reference to the accompanying drawings, in which:
Fig. 1 is an isometric view of a medical device according to an embodiment of the invention in a collapsed configuration;
Fig. 2 is an isometric view of the medical device of Fig. 1 in an expanded configuration;
Fig. 2(a) is an isometric view of the medical device of Fig. 1 in the expanded configuration;
Fig. 3 is a cross-sectional side view of a blood vessel;
Figs. 4 to 6 are cross-sectional side views illustrating movement of a stent from a delivery configuration to an intermediate configuration in the blood vessel of Fig. 3;
Figs. 7 to 10 are cross-sectional side views illustrating movement of the stent from the intermediate configuration to a deployed configuration using the medical device of Fig. 1;
Figs. 10(a) to 10(e) are cross-sectional side views illustrating movement of the stent of Figs. 4 to 10 from the delivery configuration to the deployed configuration using the medical device of Fig. 1;
Figs. 11 to 14 are cross-sectional side views illustrating movement of another stent from a delivery configuration to an intermediate configuration in the blood vessel of Fig. 3;
Figs. 15 to 18 are cross-sectional side views illustrating movement of the stent from the intermediate configuration to a deployed configuration using the medical device of Fig. 1;
Fig. 19 is an isometric view of another medical device according to an embodiment of the invention in an expanded configuration;
Fig. 20 is an isometric view of the medical device of Fig. 19 in the expanded configuration;
Fig. 21 is an end view of another medical device according to an embodiment of the invention in a non-aligned configuration;
Fig. 22 is an end view of the medical device of Fig. 21 in a semi-aligned configuration;
Fig. 23 is an end view of the medical device of Figs. 21 and 22 in an aligned configuration;
Fig. 24 illustrates an alignment system of a medical device according to an embodiment of the invention wherein the expandable element is in a non-aligned configuration;
Fig. 25 illustrates an alignment system of a medical device according to the embodiment of Figure 24 wherein the expandable element is in an aligned configuration;
Fig. 26 illustrates an alignment system of a medical device according to a further embodiment of the invention wherein the expandable element is in a non-aligned configuration;
Fig. 27 illustrates an alignment system of a medical device according to the embodiment of Figure 26 wherein the expandable element is in an aligned configuration;
Fig. 28(a) illustrates a fluoroscope and 28(b) is an elevation view of a medical device having notched portions to aid alignment, when viewed with the fluoroscope;
Fig. 29(a) illustrates a fluoroscope and 29(b) is a plan view of the medical device of Figure 28(b) having notched portions to aid alignment, when viewed with the fluoroscope;
Figs. 30(a)-(c) illustrate a stent and expandable element with a single bend in one direction, provided with alignment means; and
Figs. 31 (a)-(c) illustrate a stent and expandable element with three dimensional curvature, provided with alignment means.

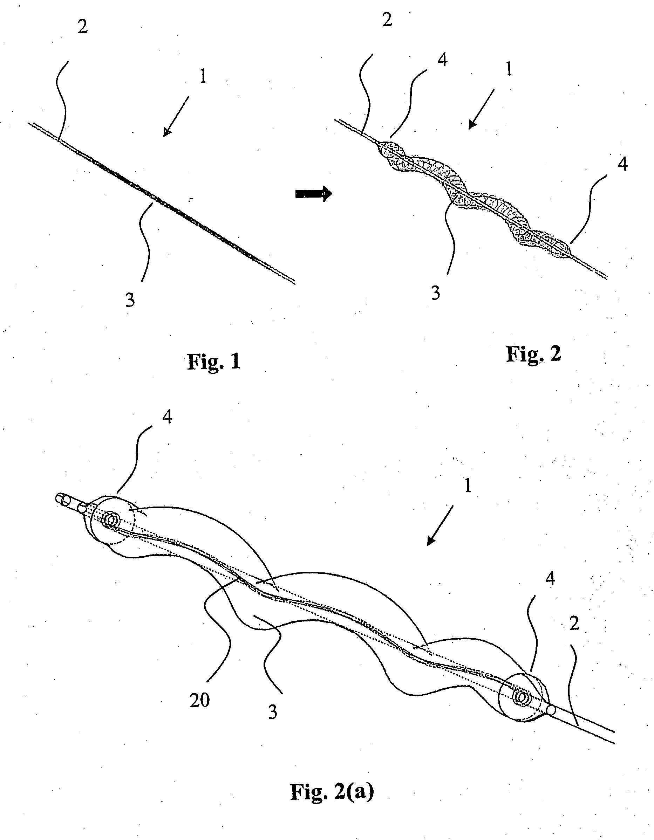

Referring to the drawings, and initially to Figs. 1 to 2(a) thereof, there is illustrated a medical system according to an embodiment of the invention. In this case the medical system comprises a stent deployment system.

The stent deployment system comprises a stent deployment device 1, and a stent suitable for deployment in a blood vessel 5 to support at least part of the internal wall of the blood vessel 5.

The stent deployment device 1 comprises an elongate catheter shaft 2, and a single expandable element 3 for location in the blood vessel 5. The expandable element 3 is mounted on the catheter shaft 2.

In this case the expandable element 3 comprises an inflatable balloon. The inflatable balloon 3 is movable between a collapsed configuration (Fig .1) and an expanded configuration (Fig. 2). In the collapsed configuration the longitudinal axis of the balloon 3 is straight, and the balloon 3 is cylindrically shaped. In the expanded configuration part of the longitudinal axis of the balloon 3 is curved in three-dimensional space, and part of the balloon 3 is helically shaped. The balloon has at each end an end part 4 in which the helical angle of the longitudinal axis of the end part increases in a direction away from the end of the balloon.

The balloon 3 is suitable for expanding the stent.

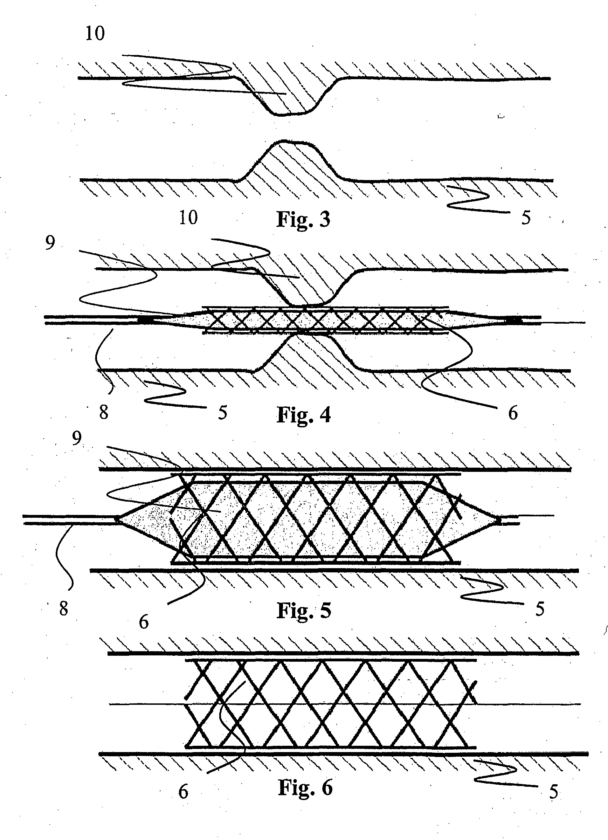

The stent may be a balloon expandable stent 6, as illustrated in Figs. 3 to 10(e). Alternatively the stent maybe a self-expanding stent 7, as illustrated in Figs. IT to 18.

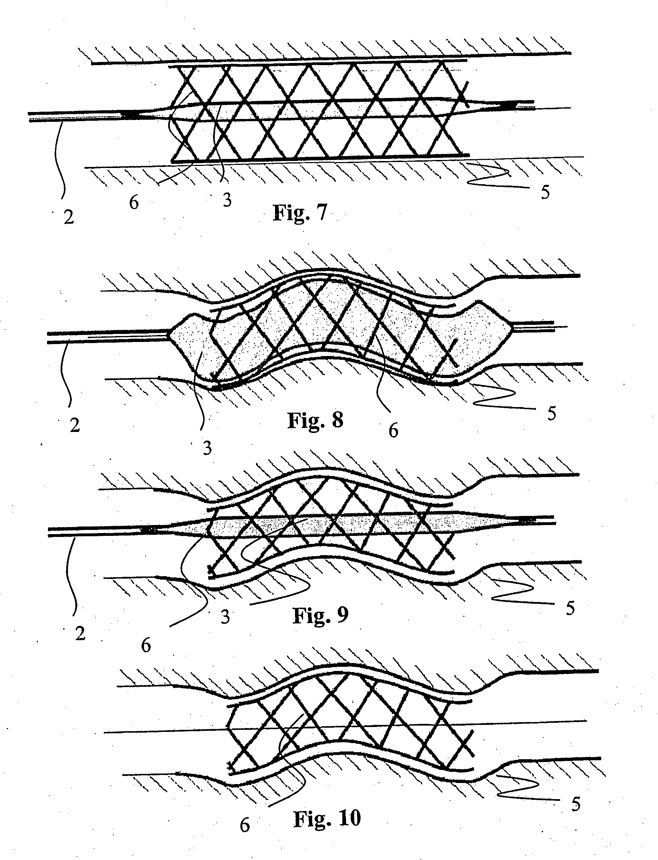

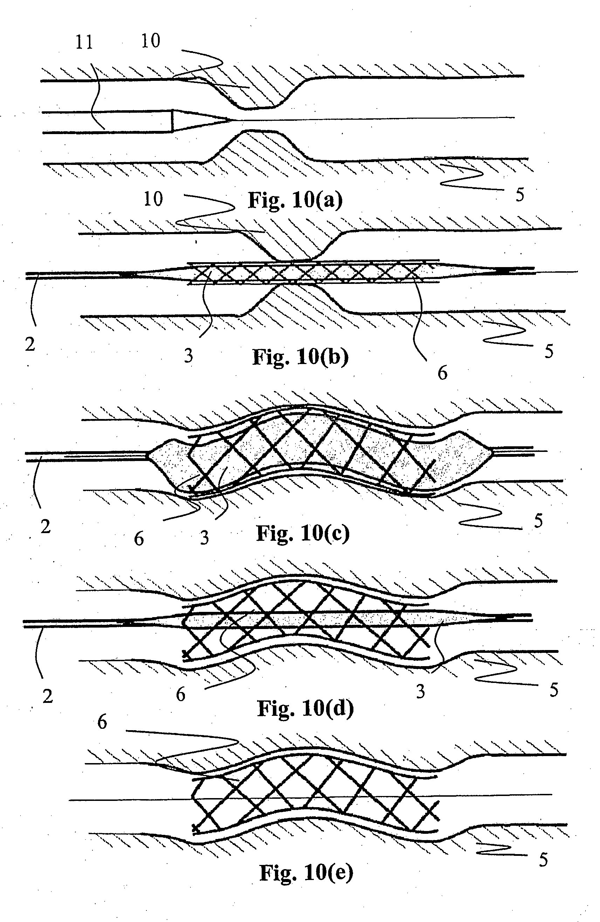

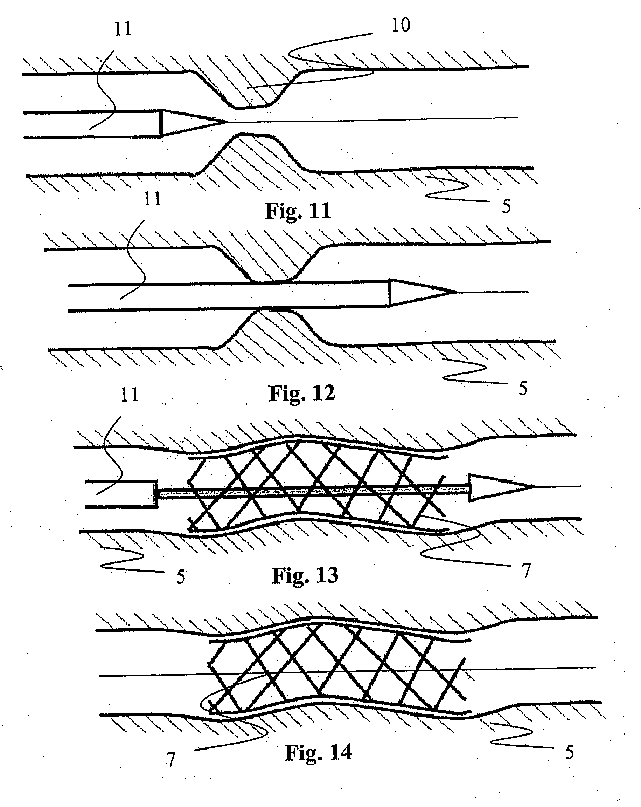

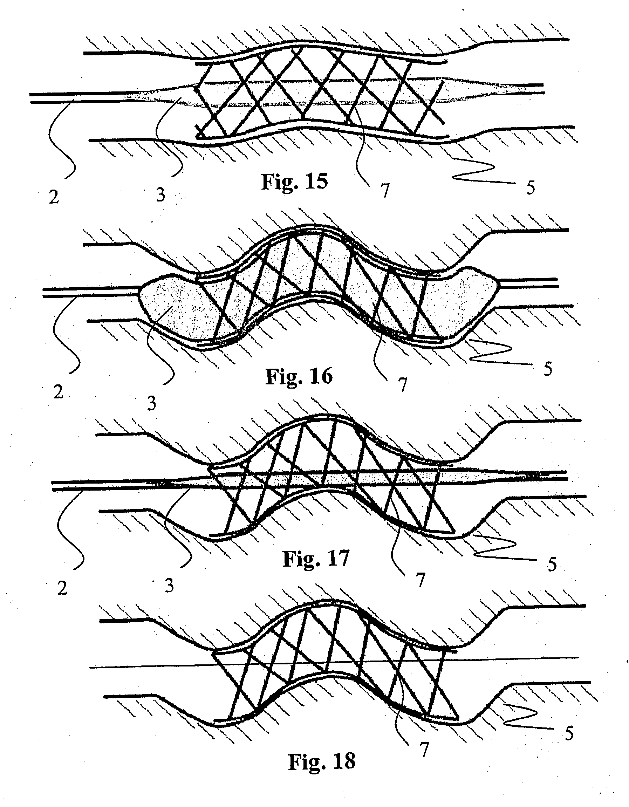

The stent 6, 7 may be movable from a collapsed delivery configuration (Figs. 4 and 12) to a partially expanded intermediate configuration (Fig. 6 and 14), and subsequently from the intermediate configuration to a fully expanded deployed configuration (Figs. 10 and 18). In the delivery configuration the longitudinal axis of the stent 6, 7 is straight. In the deployed configuration the longitudinal axis of the stent 6, 7 is curved in three-dimensional space.

The longitudinal axis of the stent 6 may be straight in the intermediate configuration (Fig. 6). The balloon 3 is suitable for expanding the stent 6 having the straight longitudinal axis. In this case the balloon 3 expands the stent 6 to ensure that in the deployed configuration the longitudinal axis of the stent 6 is curved in three-dimensional space.

Alternatively the longitudinal axis of the stent 7 may be curved in three-dimensional space in the intermediate configuration (Fig. 14). The balloon 3 is suitable for expanding the stent 7 having the longitudinal axis which curves in three-dimensional space. In this case the balloon 3 expands the stent 7 to ensure that in the deployed configuration the stent 7 is bedded into the internal wall of the blood vessel 5 and the stent 7 has taken up the fully deployed three-dimensional curve. In addition the balloon 3 expands the stent 7 to ensure that the stent amplitude ratio is such that it is not possible for blood to flow through the blood vessel 5 or the stent 7 without undergoing swirling. The amplitude ratio is the ratio of the amplitude of the helical longitudinal axis of the stent to the internal diameter of the stent.

The balloon 3 is inflated to expand the stent 7 enhancing its helical structure, increasing the amplitude ratio, reducing the pitch, anchoring the stent 7 in place, and ensuring good wall apposition.

The stent 6 may be movable directly from the collapsed delivery configuration (Fig. 10(b)) to the fully expanded deployed configuration (Fig. 10(c)) without any intermediate configuration. In the delivery configuration the longitudinal axis of the stent 6 is straight. In the deployed configuration the longitudinal axis of the stent 6 is curved in three-dimensional space.

The balloon 3 is suitable for expanding the stent 6 having the straight longitudinal axis. In this case the balloon 3 expands the stent 6 to ensure that in the deployed configuration the longitudinal axis of the stent 6 is curved in three-dimensional space.

In each case the balloon 3 in the expanded configuration exerts force on the stent 6, 7. In the deployed configuration the stent 6, 7 in turn exerts force on the internal wall of the blood vessel 5 causing the longitudinal axis of the blood vessel 5 to curve in three-dimensional space (Figs. 8 and 16).

As illustrated in Fig. 2(a), the stent deployment device 1 also comprises visualisation means to align the balloon 3 with the stent 6, 7. In this case the visualisation means comprises a marker 20 on the balloon 3. The marker 20 extends circumferentially and longitudinally along the balloon 3 in a helix or spiral. The marker 20 may be aligned with the helical shape of the stent 6, 7.

Fig. 2(a) illustrates the helical marker 20 on the helical balloon 3.

The stent deployment device 1 may be configured for rapid exchange delivery over a guidewire or over-the-wire delivery over a guidewire.

Fig. 1 illustrates the helical balloon 3 unexpanded on the catheter 2, and Fig. 2 illustrates the helical balloon 3 expanded on the catheter 2.

In use, the stent deployment device 1 may be used to deploy the balloon expandable stent 6 in the blood vessel 5 to support at least part of the internal wall of the blood vessel 5, as illustrated in Figs. 3 to 10(e). Alternatively the stent deployment device 1 may be used to deploy the self-expanding stent 7 in the blood vessel 5 to support at least part of the internal wall of the blood vessel 5, as illustrated in Figs. 11 to 18.

In one case the balloon expandable stent 6 is arranged in the delivery configuration mounted to a delivery catheter 8. In the delivery configuration the longitudinal axis of the stent 6 is straight. The delivery catheter 8 comprises an inflatable balloon 9.

The balloon 9 is movable between a collapsed configuration (Fig .4) and an expanded configuration (Fig. 5). In the collapsed configuration the longitudinal axis of the balloon 9 is straight, and the balloon 9 is cylindrically shaped. In the expanded configuration the longitudinal axis of the balloon 9 is straight, and the balloon 9 is cylindrically shaped.

The delivery catheter 8 and the stent 6 are inserted into the blood vessel 5 and advanced until the stent 6 is located at a desired treatment site 10 (Fig. 4). The balloon 9 is inflated to move the stent 6 from the delivery configuration to the intermediate configuration (Fig. 5). The longitudinal axis of the stent 6 is straight in the intermediate configuration. The balloon 9 is deflated and the delivery catheter 8 is withdrawn (Fig. 6).

The stent deployment device 1 of the invention is then inserted into the blood vessel 5 and advanced until the balloon 3 is aligned with the stent 6 (Fig. 7). The balloon 3 is inflated to expand the stent 6 from the intermediate configuration to the deployed configuration. The balloon 3 in the expanded configuration exerts force on the stent 6 to move the stent 6 from the intermediate configuration to the deployed configuration (Fig. 8). In the deployed configuration the longitudinal axis of the stent 6 is curved in three-dimensional space. In the deployed configuration the stent 6 exerts force on the internal wall of the blood vessel 5 causing the longitudinal axis of the blood vessel 5 to curve in three-dimensional space.

The balloon 3 is deflated (Fig. 9), and the stent deployment device 1 is withdrawn from the blood vessel 5 (Fig. 10). The deployed stent 6 remains in the blood vessel 5.

Blood flowing through the three-dimensional curved blood vessel 5 undergoes a swirling action. The swirling flow of blood has been found to minimise thrombosis and platelet adhesion, and to minimise or prevent coverage of the stent 6 by ingrowth of intima. The flow pattern in the blood vessel 5 including the swirling pattern induced by the non-planar geometry of the blood vessel 5 operates to inhibit the development of vascular diseases such as thrombosis/atherosclerosis and intimal hyperplasia.

Figs. 3 to 10 illustrate the balloon expandable straight stent 6 with the 3-D balloon 3. Fig. 3 illustrates the stenosed vessel 10. Fig. 4 illustrates the crimped stent 6 on the delivery system 8. Fig. 5 illustrates the balloon 9 inflated and the stent 6 deployed. Fig. 6 illustrates the vessel patency restored. Fig. 7 illustrates the 3-D balloon catheter 1. Fig. 8 illustrates the 3-D balloon 3 inflated. Fig. 9 illustrates the 3-D balloon 3 deflated for withdrawal. Fig. 10 illustrates the stent 6 and the blood vessel 5 deformed to assume the 3-D curvature.

In another case the balloon expandable stent 6 is arranged in the delivery configuration mounted to the stent deployment device 1 of the invention. In the delivery configuration the longitudinal axis of the stent 6 is straight.

The stent deployment device 1 and the stent 6 are inserted into the blood vessel 5 and advanced until the stent 6 is located at a desired treatment site 10 (Fig. 10(b)). The balloon 3 is inflated to expand the stent 6 from the delivery configuration to the deployed configuration (Fig. 10(c)). The balloon 3 in the expanded configuration exerts force on the stent 6 to move the stent 6 from the delivery configuration to the deployed configuration. In the deployed configuration the longitudinal axis of the stent 6 is curved in three-dimensional space. In the deployed configuration the stent 6 exerts force on the internal wall of the blood vessel 5 causing the longitudinal axis of the blood vessel 5 to curve in three-dimensional space.

The balloon 3 is deflated (Fig. 10(d)), and the stent deployment device 1 is withdrawn from the blood vessel 5 (Fig. 10(e)). The deployed stent 6 remains in the blood vessel 5.

Blood flowing through the three-dimensional curved blood vessel 5 undergoes a swirling action. The swirling flow of blood has been found to minimise thrombosis and platelet adhesion, and to minimise or prevent coverage of the stent 6 by ingrowth of intima. The flow pattern in the blood vessel 5 including the swirling pattern induced by the non-planar geometry of the blood vessel 5 operates to inhibit the development of vascular diseases such as thrombosis/atherosclerosis and intimal hyperplasia.

Figs. 10(a) to 10(e) illustrate the straight stent 6 crimped onto the helical balloon 3.

In the case of the self-expanding stent 7, the stent 7 is arranged in the delivery configuration constrained within a delivery sheath 11. In the delivery configuration the longitudinal axis of the stent 7 is straight.

The delivery sheath 11 and the stent 7 are inserted into the blood vessel 5 and advanced until the stent 7 is located at the desired treatment site 10 (Figs. 11 and 12). The sheath 11 is retracted to enable the stent 7 to move from the delivery configuration to the intermediate configuration (Fig. 13). The longitudinal axis of the stent 7 is curved in three-dimensional space in the intermediate configuration. In the intermediate configuration the stent 7 exerts force on the internal wall of the blood vessel 5 causing the longitudinal axis of the blood vessel 5 to curve in three-dimensional space. The delivery sheath 11 is withdrawn (Fig. 14).

The stent deployment device 1 of the invention is then inserted into the blood vessel 5 and advanced until the balloon 3 is aligned with the stent 7 (Fig. 15). The balloon 3 is inflated to expand the stent 7 from the intermediate configuration to the deployed configuration. The balloon 3 in the expanded configuration exerts force on the stent 7 to move the stent 7 from the intermediate configuration to the deployed configuration (Fig. 16). In the deployed configuration the longitudinal axis of the stent 7 is further curved in three-dimensional space. In the deployed configuration the stent 7 exerts further force on the internal wall of the blood vessel 5 causing the longitudinal axis of the blood vessel 5 to curve further in three-dimensional space.

The balloon 3 is deflated (Fig. 17), and the stent deployment device 1 is withdrawn from the blood vessel 5 (Fig. 18).

Blood flowing through the three-dimensional curved blood vessel 5 undergoes a swirling action. The swirling flow of blood has been found to minimise thrombosis and platelet adhesion, and to minimise or prevent coverage of the stent 7 by ingrowth of intima. The flow pattern in the blood vessel 5 including the swirling pattern induced by the non-planar geometry of the blood vessel 5 operates to inhibit the development of vascular diseases such as thrombosis/atherosclerosis and internal hyperplasia.

Figs. 11 to 18 illustrate the 3-D stent 7 with the 3-D balloon 3. Fig. 11 illustrates the stenosed vessel 10 and the stent delivery system 11. Fig. 13 illustrates the 3-D stent 7 deployed. Fig. 14 illustrates the blood vessel 5 remodelled to take the 3-D curvature. Fig. 15 illustrates the balloon catheter 1. Fig. 17 illustrates the 3-D balloon 3 deflated for withdrawal. Fig. 18 illustrates the balloon 3 withdrawn, and the stent 7 with maximised 3-D curvature in place.

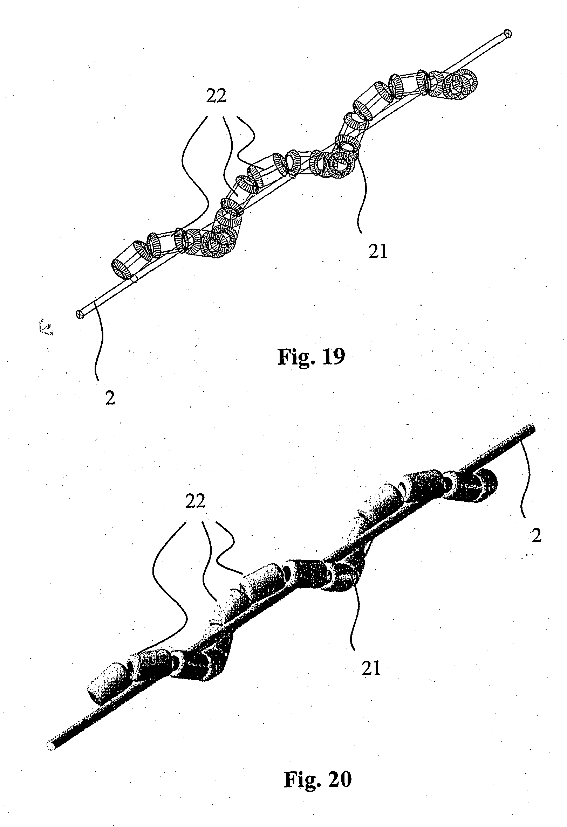

It will be appreciated that the expandable element 21 may comprise a plurality of separate expandable members 22, as illustrated in Figs. 19 and 20. In the expanded configuration the overall longitudinal axis of the expandable element 21 is curved in three-dimensional space, and the expandable element 21 is helically shaped overall. In the expanded configuration the longitudinal axis of each expandable member 22 is straight, and each expandable member 22 is cylindrically shaped. The expandable element 21 has a piecewise three dimensional curved shape. Figs. 19 and 20 illustrate the piecewise balloon 21 comprised of the plurality of straight balloons 22.

It will also be appreciated that the expandable element may have alternative shapes, for example in the expanded configuration part of the expandable element may be spiral shaped. The pitch of the spiral may be constant along the length of the expandable element, or may vary along the length of the expandable element.

It will further be appreciated that the stent deployment device may comprise a variety of possible visualisation means to align the expandable element with a stent.

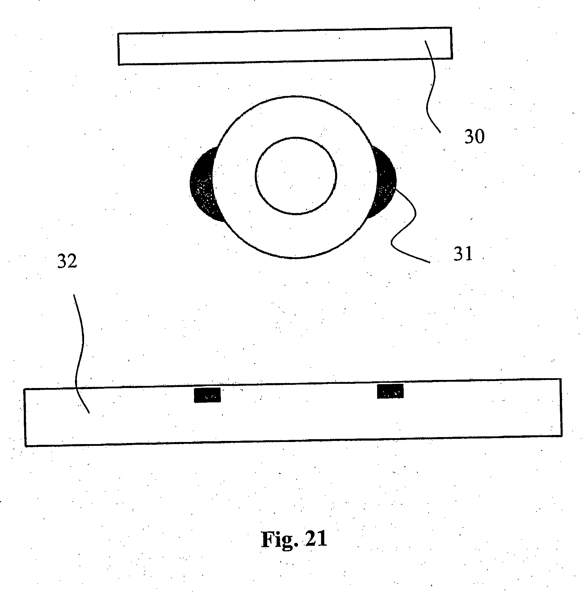

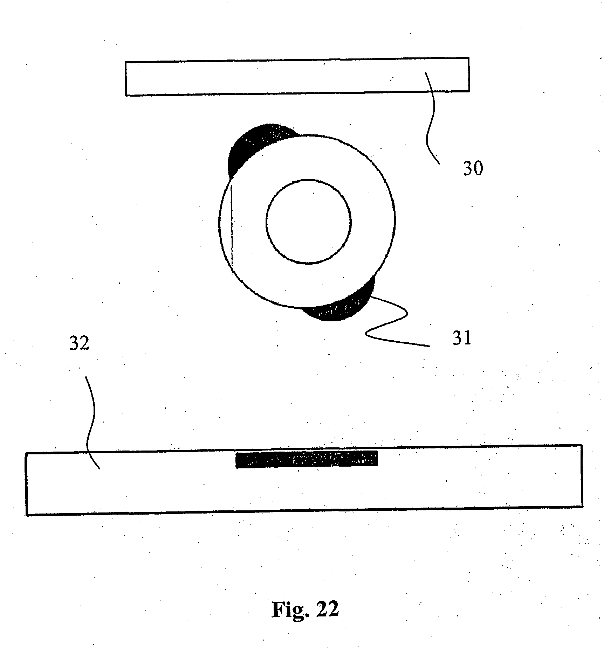

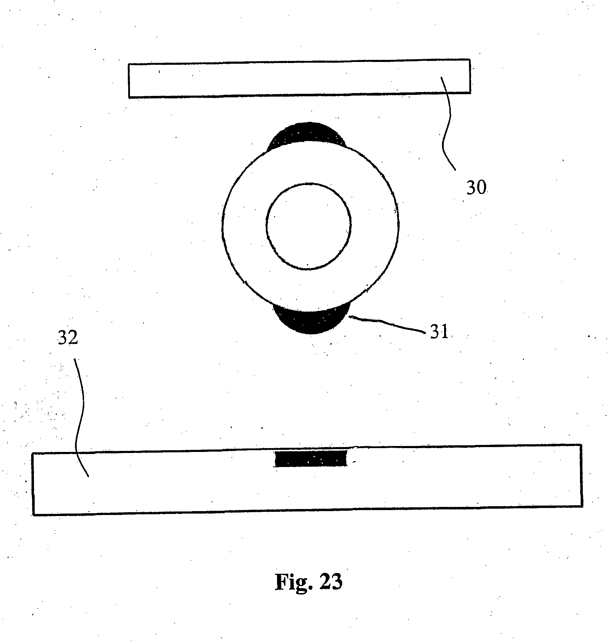

For example Figs. 21 - 23 illustrate visualisation means comprising alignment markers. The system utilises fluoroscopy, and includes an x-ray source 30, markers 31 located on an expandable element and imaging media 32. A stent (not illustrated) is located around the expandable element. The markers comprise a radiopaque material. Figure 21 shows the expandable element before alignment. The extent of alignment is indicated by the image produced by imaging media 32. This embodiment has been devised so that the image shown in Figure 21, i.e. two small marks, indicates an unaligned state. Figure 22 illustrates the expandable element having been rotated towards an aligned position, as indicated by the single wide mark on the imaging media 32. Figure 23 illustrates the expandable element aligned with the stent, as indicated by the single narrower mark on the imaging media 32.

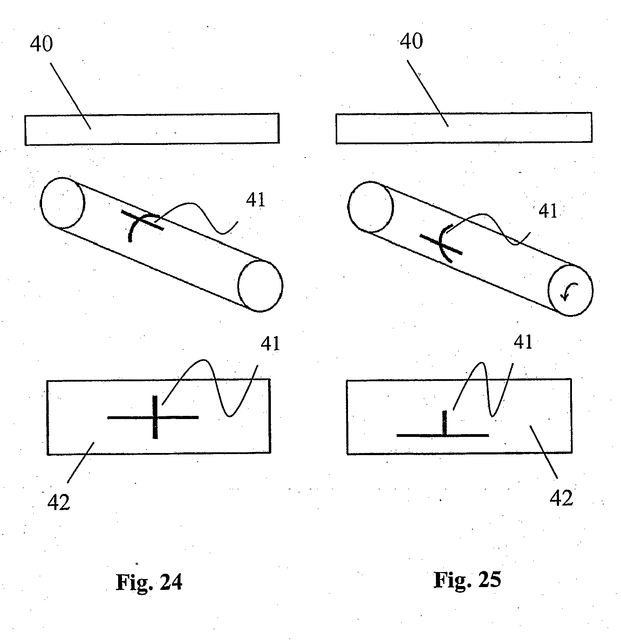

Figs. 24 and 25 illustrate a further embodiment comprising a cross shaped alignment marker 41 on an expandable element. As shown in Figure 24, when viewed utilising fluroscopy on the imaging media 42 using x-ray source 40, the marker 41 appears as a cross in the first orientation of the expandable element. When the expandable element is rotated into the correct alignment. as shown in Figure 25, the marker 41 will appear as a T-shape on the imaging media 42.

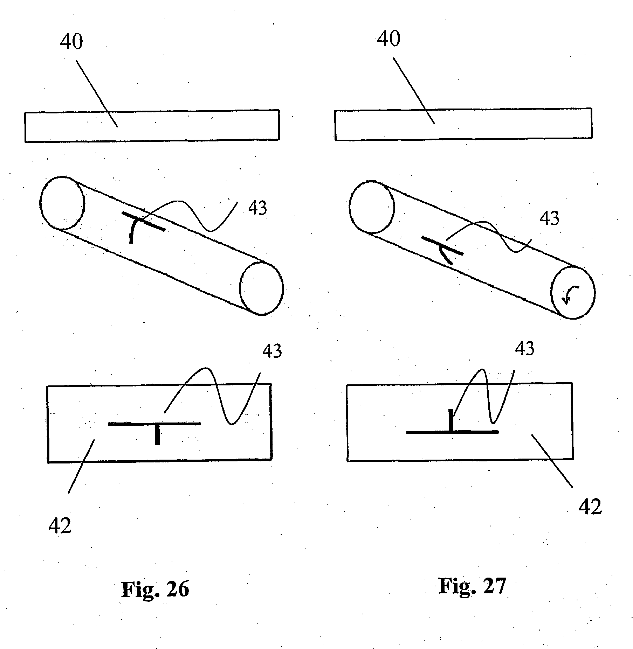

Figs. 26 and 27 illustrate a further embodiment comprising a T shaped alignment marker 43 on an expandable element. As shown in Figure 26, when viewed on the imaging media 42 using x-ray source 40 the marker 43 appears as a T in the first orientation of the expandable element. When the expandable element is rotated into the correct alignment as shown in Figure 27, the marker 41 will appear to have been rotated by 180 degrees.

It will also be appreciated that the medical system according to the invention may comprise a dilation system. The dilation system comprises a dilation device for dilating a blood vessel. The dilation device comprises the elongate catheter shaft, and the single expandable element for location in the blood vessel. In the expanded configuration the expandable element contacts the internal wall of the blood vessel directly.

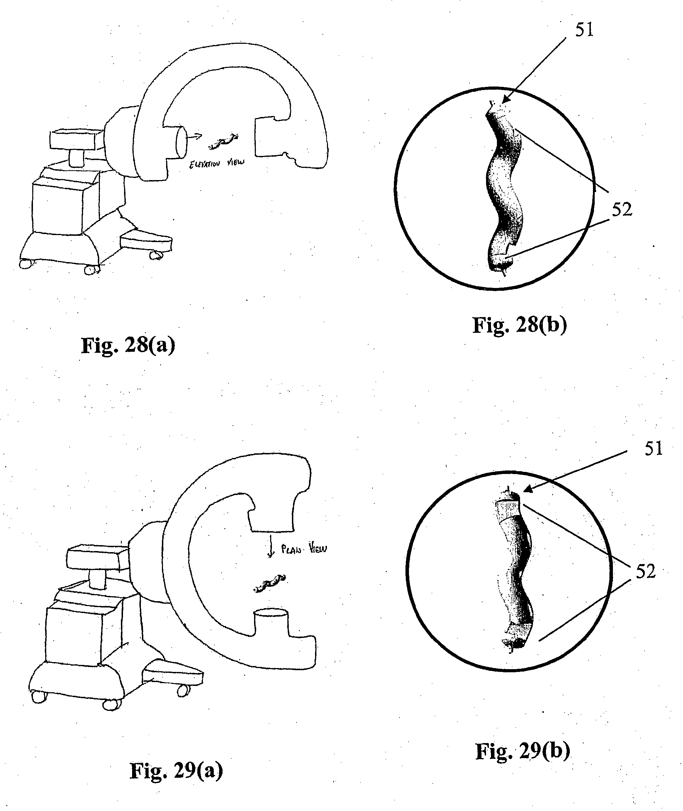

Figures 28 to 31 illustrate an alternative means for aligning the expandable element with a stent, or, in the case of a dilation system, directly with the blood vessel lumen.

Figures 28(b) and 29(b) illustrate an expandable element 51 in vivo in a body (not illustrated) including alignment means. Figure 28(b) is an elevation (lateral) projection as viewed using the C-arm of a fluoroscope as illustrated in Figure 28(a). Figure 29(b) is a plan (anterior-posterior) projection as viewed using the C-arm of a fluoroscope as illustrated in Figure 29(a). As can be seen, the expandable element 51 has a generally cylindrical cross section, and comprises a generally flat notch section 52 near each end.

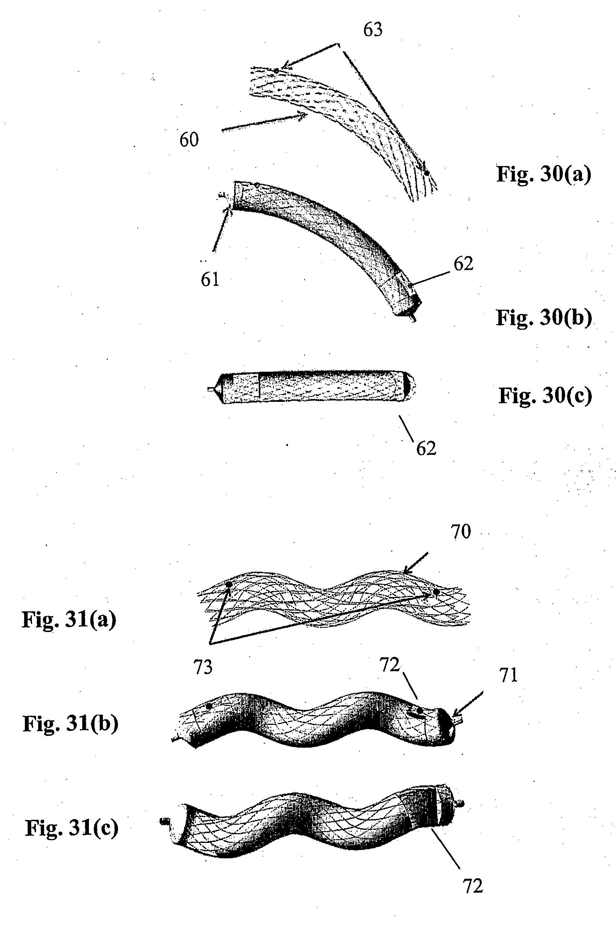

In use, when the expandable element 51 is inflated with saline or a contrast enhancing medium (radiopaque dye), the flat notch sections 52 will be visible when viewered using fluoroscopy. Using the capability of the c-arm on a fluoroscope (see Figures 28(a) and 29(a)), it is possible to image the expandable element 51 in two orthogonal views to aid positioning, e.g. in elevation and plan views as shown in Figures 28(b) and 29(b) respectively. The orientation of the expandable element 51 can be adjusted in vivo to the correct position (i.e. in alignment with the curvature of the stent or blood vessel lumen) based on the orientation of the flat notched sections 52 with respect to another reference point, such as a marker on a self expanding stent, or the curvature of the stent / vessel lumen. The use of a marker is described further with reference to Figures 30(a)-(c) and 31(a)-(c).

Figures 30(a)-(c) illustrate a stent 60 and expandable element 61 having a single bend in one direction (i.e. a centreline curved in one dimension). Figure 30(a) illustrates the stent 60. This comprises a pair of diametrically opposed radiopaque markers 63 near each end (only one of each pair is illustrated). Figures 30(b) and (c) show the expandable element 61 inserted in the stent 60; Figure 30(b) being an elevation view and Figure 30(c) being a plan view. As can be seen, the expandable element 61 comprises flat notched sections 62 as described previously above. The radiopaque markers 63 are positioned so as to match up with the flat notched sections 62 when the expandable element is correctly aligned.

Thus, in use, fluoroscopy can be used to view the expandable element 61, for example in two orthogonal directions such as elevation and plan view, in order to align the notches 62 with the radiopaque markers 63 and thus position the expandable element 61 correctly.

Figures 31(a)-(c) are similar to Figures 30(a)-(c) but illustrate a stent 70 and expandable element 71 having longitudinal axes curved in three-dimensional space. Figure 31(a) illustrates the stent 70. This comprises a pair of diametrically opposed radiopaque markers 73 near each end (only one of each pair is illustrated). Figures 31(b) and (c) show the expandable element 71 inserted in the stent 70; Figure 31(b) being an elevation view and Figure 31 (c) being a plan view. As can be seen, the expandable element 71 comprises flat notched sections 72 as described previously above. The radiopaque markers 73 are positioned so as to match up with the flat notched sections 72 when the expandable element is correctly aligned.

Thus, as with the embodiment of Figure 30 above, in use, fluoroscopy can be used to view the expandable element 71, for example in two orthogonal directions such as elevation and plan view. The notches 72 can then be aligned with the radiopaque markers 73 in order to position the expandable element 71 correctly with respect to the curvature of the stent 70.

The invention is not limited to the embodiments hereinbefore described, with reference to the accompanying drawings, which may be varied in construction and detail.

## Claims

1. A medical device comprising:-
a balloon (3, 51, 61, 71) for location in a blood vessel, the balloon having at each end an end part (4);
the balloon being inflatable between a collapsed configuration and an expanded configuration; wherein
in the expanded configuration at least part of the longitudinal axis of the balloon is curved in three-dimensional space; **characterised in that**:
in the expanded configuration at least part of the balloon is substantially helically shaped; and wherein a longitudinal axis of each end part of the balloon has a helical angle that varies along the length of the end part and which increases in a direction away from the end of the balloon.

2. A device as claimed in claim 1 wherein in the expanded configuration the balloon (3, 51, 61, 71) is configured to exert force on the internal wall of a blood vessel (5) causing the longitudinal axis of the blood vessel to curve in three-dimensional space.

3. A device as claimed in claim 1 or 2 wherein the device comprises a single balloon (3, 51, 61, 71) or a plurality of separate balloons (22);
wherein preferably the device comprises an elongate element (2) upon which the balloon(s) is mounted.

4. A device as claimed in any of claims 1 to 3 wherein in the collapsed configuration at least part of the longitudinal axis of the balloon (3, 51, 61, 71) is substantially straight;
wherein preferably in the collapsed configuration at least part of the balloon is substantially cylindrically shaped.

5. A device as claimed in any preceding claim wherein the balloon (3, 51, 61, 71) is configured to at least partially expand a stent (6, 7, 60, 70);
wherein preferably in the expanded configuration the balloon is configured to exert force via a stent on the internal wall of a blood vessel;
wherein preferably the device comprises a stent deployment device (1);
wherein preferably the balloon is configured to expand a stent having a longitudinal axis which curves in three-dimensional space (7) or a longitudinal axis which is substantially straight (6).

6. A device as claimed in any of claims 1 to 4 wherein in the expanded configuration the balloon (3, 51) is configured to contact the internal wall of a blood vessel (5) directly;
wherein preferably the device comprises a dilation device.

7. A device as claimed in any preceding claim wherein the device comprises means to align the balloon (3, 51, 61, 71) with a stent (6, 7, 60, 70) or blood vessel (5).

8. A device as claimed in claim 7 wherein the alignment means extends circumferentially and/or longitudinally along at least part of the balloon (3);
wherein preferably the alignment means comprises means (20, 31) to visualise the balloon, preferably one or more markers (20, 31) on the balloon, which preferably comprise radiopaque material.

9. A device as claimed in claim 7 wherein said means is provided by the balloon (51, 61, 71) having a varying cross section;
wherein preferably the variation is such that a projection view of the cross-section varies as the balloon is axially rotated;
wherein preferably the balloon has a notched cross section, the notches (52, 62, 72) comprising substantially flat portions recessed in the outer surface of the balloon.

10. A medical system comprising:-
a medical device as claimed in any of claims 1 to 5, 7 or 8; and
a stent (6, 7, 60, 70) suitable for deployment in a blood vessel to support at least part of the internal wall of the blood vessel.

11. A medical system as claimed in claim 10 when dependent on claim 9,
wherein the stent (60, 70) is provided with radiopaque markers (63, 73) located so as to be positioned in a predetermined way relative to the varying cross-section (62, 72) when the balloon (61, 71) is correctly aligned within the stent;
wherein preferably at least two pairs of radiopaque markers (63, 73) are provided, each pair comprising two markers arranged diametrically opposite around the circumference of the stent (60, 70).

12. A system as claimed in claim 10 or 11, wherein the stent (6, 7, 60, 70) is movable between a delivery configuration and a deployed configuration;
wherein preferably the stent is collapsed in the delivery configuration;
wherein preferably the stent is expanded in the deployed configuration.

13. A system as claimed in claim 12 wherein in the delivery configuration at least part of the longitudinal axis of the stent (6, 7, 60, 70) is substantially straight;
wherein preferably in the deployed configuration at least part of the longitudinal axis of the stent is curved in three-dimensional space, the stent preferably being configured to exert force on the internal wall of a blood vessel (5) causing the longitudinal axis of the blood vessel to curve in three-dimensional space.

14. A system as claimed in claim 11 or 12 wherein the stent (6, 7, 60, 70) is movable between the delivery configuration and an intermediate configuration, and between the intermediate configuration and the deployed configuration;
wherein preferably the stent is at least partially expanded in the intermediate configuration;
wherein preferably in the intermediate configuration at least part of the longitudinal axis of the stent is substantially straight or is curved in three-dimensional space.

15. A system as claimed in any of claims 10 to 14 wherein in the expanded configuration of the balloon (3, 51, 61, 71), the balloon is configured to exert force via the stent (6, 7, 60, 70) on the internal wall of a blood vessel (5) to deform the internal wall of the blood vessel such that the longitudinal axis of the blood vessel is caused to curve in three-dimensional space.

## Patentansprüche

1. Medizinisches Gerät, umfassend:
einen Ballon (3, 51, 61, 71) zur Einlage in ein Blutgefäß, wobei der Ballon an jedem Ende ein Endteil (4) aufweist;
wobei der Ballon zwischen einer zusammengezogenen und einer erweiterten Konfiguration aufblasbar ist; wobei
in der erweiterten Konfiguration mindestens ein Teil der Längsachse des Ballons in dreidimensionalem Raum gekrümmt ist, **dadurch gekennzeichnet,**
**dass** in der erweiterten Konfiguration mindestens ein Teil des Ballons im Wesentlichen schraubenförmig ist; und wobei eine Längsachse jedes Endteils des Ballons einen schraubenförmigen Winkel aufweist, der über die Länge des Endteils variiert und mit zunehmendem Abstand zum Ende des Ballons zunimmt.

2. Gerät nach Anspruch 1, wobei in der erweiterten Konfiguration der Ballon (3, 51, 61, 71) derart konfiguriert ist, dass er auf die Innenwand eines Blutgefäßes (5) eine Kraft ausübt, wodurch die Längsachse des Blutgefäßes in dreidimensionalem Raum gekrümmt wird.

3. Gerät nach Anspruch 1 oder 2, wobei das Gerät einen einzelnen Ballon (3, 51, 61, 71) oder eine Mehrzahl getrennter Ballons (22) umfasst;
wobei das Gerät vorzugsweise ein längliches Element (2) umfasst, auf dem der mindestens eine Ballon montiert ist.

4. Gerät nach einem der Ansprüche 1 - 3, wobei in der zusammengezogenen Konfiguration mindestens ein Teil der Längsachse des Ballons (3, 51, 61, 71) im Wesentlichen geradlinig ist;
wobei in der erweiterten Konfiguration mindestens ein Teil des Ballons vorzugsweise im Wesentlichen zylindrisch.

5. Gerät nach einem der vorstehenden Ansprüche, wobei der Ballon (3, 51, 61, 71) derart konfiguriert ist, dass er einen Stent (6, 7, 60, 70) mindestens teilweise erweitert;
wobei der Ballon in der erweiterten Konfiguration vorzugsweise zur Ausübung einer Kraft auf die Innenwand eines Blutgefäßes über einen Stent konfiguriert ist;
wobei das Gerät vorzugsweise ein Stent-Einsatzgerät (1) umfasst;
wobei der Ballon vorzugsweise derart konfiguriert ist, dass er einen Stent erweitert, der eine in dreidimensionalem Raum gekrümmte Längsachse (7) oder eine im Wesentlichen geradlinige Längsachse (6) aufweist.

6. Gerät nach einem der Ansprüche 1 - 4, wobei der Ballon (3, 51) in der erweiterten Konfiguration zum unmittelbaren Kontaktieren der Innenwand eines Blutgefäßes (5) konfiguriert ist;
wobei das Gerät vorzugsweise ein Dilatationsgerät umfasst.

7. Gerät nach einem der vorstehenden Ansprüche, wobei das Gerät Mittel zur Ausrichtung des Ballons (3, 51, 61, 71) mit einem Stent (6, 7, 60, 70) oder Blutgefäß (5) umfasst.

8. Gerät nach Anspruch 7, wobei sich die Ausrichtungsmittel in Umfangs- und/oder Längsrichtung entlang mindestens einem Teil des Ballons (3) erstreckt;
wobei die Ausrichtungsmittel vorzugsweise Mittel (20, 31) zur Visualisierung des Ballons, vorzugsweise eine oder mehrere Markierungen (20, 31) am Ballon umfassen, die vorzugsweise röntgendichtes Material umfassen.

9. Gerät nach Anspruch 7, wobei die Mittel dadurch vorgesehen sind, dass der Ballon (51, 61, 71) einen variierenden Querschnitt aufweist;
wobei die Variation vorzugsweise derart ist, dass die Projektionsansicht des Querschnitts mit der axialen Rotation des Ballons variiert;
wobei der Ballon vorzugsweise einen ausgeklinkten Querschnitt aufweist; wobei die Kerben (52, 62, 72) in der Außenfläche des Ballons vertiefte, im Wesentlichen flache Abschnitte umfassen.

10. Medizinisches System, umfassend:
ein medizinisches Gerät nach einem der Ansprüche 1 - 5, 7 oder 8; und
einen Stent (6, 7, 60, 70), der zum Einsatz in einem Blutgefäß geeignet ist, um mindestens einen Teil der Innenwand des Blutgefäßes abzustützen.

11. Medizinisches System nach Anspruch 10, soweit dieser vom Anspruch 9 abhängt,
wobei der Stent (60, 70) mit röntgendichten Markierungen (63, 73) versehen ist, die derart angeordnet sind, dass sie relativ zum variierenden Querschnitt (62, 72) auf vorbestimmte Weise positioniert sind, wenn der Ballon (61, 71) im Stent richtig ausgerichtet ist;
wobei vorzugsweise mindestens zwei Paare röntgendichter Markierungen (63, 73) vorgesehen sind, wobei jedes Paar zwei um den Umfang des Stents (60, 70) einander diametral gegenüberliegende Markierungen umfasst.

12. System nach Anspruch 10 oder 11, wobei der Stent (6, 7, 60, 70) zwischen einer Einführungskonfiguration und einer Einsatzkonfiguration beweglich ist;
wobei der Stent in der Einführungskonfiguration vorzugsweise zusammengezogen ist;
wobei der Stent in der Einsatzkonfiguration vorzugsweise erweitert ist.

13. System nach Anspruch 12, wobei in der Einsatzkonfiguration mindestens ein Teil der Längsachse des Stents (6, 7, 60, 70) im Wesentlichen geradlinig ist;
wobei in der Einsatzkonfiguration mindestens ein Teil der Längsachse des Stents in dreidimensionalem Raum gekrümmt wird, und wobei der Stent vorzugsweise derart konfiguriert ist, dass er eine Kraft auf die Innenwand eines Blutgefäßes (5) ausübt, wodurch die Längsachse des Blutgefäßes in dreidimensionalem Raum gekrümmt wird.

14. System nach Anspruch 11 oder 12, wobei der Stent (6, 7, 60, 70) zwischen der Einführungskonfiguration und der Einsatzkonfiguration und zwischen der Zwischenkonfiguration und der Einsatzkonfiguration beweglich ist;
wobei der Stent in der Zwischenkonfiguration vorzugsweise mindestens teilweise erweitert ist;
wobei in der erweiterten Konfiguration vorzugsweise mindestens ein Teil der Längsachse des Stents im Wesentlichen geradlinig oder in dreidimensionalem Raum gekrümmt ist.

15. System nach einem der Ansprüche 10 - 14, wobei in der erweiterten Konfiguration des Ballons der Ballon (3, 51, 61, 71) derart konfiguriert ist, dass er über den Stent (6, 7, 60, 70) auf die Innenwand eines Blutgefäßes (5) eine Kraft ausübt, um die Innenwand des Blutgefäßes derart zu verformen, dass die Längsachse des Blutgefäßes in dreidimensionalem Raum gekrümmt wird.

## Revendications

1. Dispositif médical comprenant :
un ballonnet (3, 51, 61, 71) destiné à être placé dans un vaisseau sanguin, le ballonnet ayant à chaque extrémité une partie d'extrémité (4) ;
le ballonnet étant gonflable entre une configuration affaissée et une configuration dilatée ; dans lequel
dans la configuration dilatée, au moins une partie de l'axe longitudinal du ballonnet est incurvée dans l'espace tridimensionnel ; **caractérisé en ce que**
dans la configuration dilatée, au moins une partie du ballonnet est sensiblement de forme hélicoïdale ; et dans lequel un axe longitudinal de chaque partie d'extrémité du ballonnet présente un angle hélicoïdal qui varie le long de la longueur de la partie d'extrémité et qui augmente dans une direction s'écartant de l'extrémité du ballonnet.

2. Dispositif selon la revendication 1, dans lequel dans la configuration dilatée, le ballonnet (3, 51, 61, 71) est configuré pour exercer une force sur la paroi interne d'un vaisseau sanguin (5), amenant l'axe longitudinal du vaisseau sanguin à s'incurver dans l'espace tridimensionnel.

3. Dispositif selon la revendication 1 ou 2, le dispositif comprenant un seul ballonnet (3, 51, 61, 71) ou une pluralité de ballonnets séparés (22) ;
dans lequel le dispositif comprend de préférence un élément allongé (2) sur lequel est/sont monté(s) le (s) ballonnet(s).

4. Dispositif selon l'une quelconque des revendications 1 à 3, dans lequel dans la configuration affaissée, au moins une partie de l'axe longitudinal du ballonnet (3, 51, 61, 71) est sensiblement rectiligne ;
dans lequel dans la configuration affaissée, au moins une partie du ballonnet est de préférence de forme sensiblement cylindrique.

5. Dispositif selon l'une quelconque des revendications précédentes, dans lequel le ballonnet (3, 51, 61, 71) est configuré pour dilater au moins partiellement un stent (6, 7, 60, 70) ;
dans lequel dans la configuration dilatée, le ballonnet est de préférence configuré pour exercer une force via un stent sur la paroi interne d'un vaisseau sanguin ;
dans lequel le dispositif comprend de préférence un dispositif de déploiement de stent (1) ;
dans lequel le ballonnet est de préférence configuré pour dilater un stent ayant un axe longitudinal qui s'incurve dans l'espace tridimensionnel (7) ou un axe longitudinal qui est sensiblement rectiligne (6).

6. Dispositif selon l'une quelconque des revendications 1 à 4, dans lequel la configuration dilatée du ballonnet (3, 51) est configurée pour entrer en contact avec la paroi interne d'un vaisseau sanguin (5) directement ;
dans lequel le dispositif comprend de préférence un dispositif de dilation.

7. Dispositif selon l'une quelconque des revendications précédentes, dans lequel le dispositif comprend des moyens pour aligner le ballonnet (3, 51, 61, 71) avec un stent (6, 7, 60, 70) ou un vaisseau sanguin (5).

8. Dispositif selon la revendication 7, dans lequel les moyens d'alignement s'étendent de manière circonférentielle et/ou longitudinale le long d'au moins une partie du ballonnet (3) ;
dans lequel les moyens d'alignement comprennent de préférence des moyens (20, 31) pour visualiser le ballonnet, de préférence, un ou plusieurs marqueurs (20, 31) sur le ballonnet, qui comprennent de préférence un matériau radio-opaque.

9. Dispositif selon la revendication 7, dans lequel lesdits moyens sont fournis par le ballonnet (51, 61, 71) ayant une section transversale variable ;
dans lequel la variation est de préférence telle qu'une vue en projection de la section transversale varie au fur et à mesure que le ballonnet est tourné axialement ;
dans lequel le ballonnet présente de préférence une section transversale entaillée, les entailles (52, 62, 73) comprenant des parties sensiblement plates en retrait dans la surface externe du ballonnet.

10. Système médical comprenant :
un dispositif médical selon l'une quelconque des revendications 1 à 5, 7 ou 8 ; et
un stent (6, 7, 60, 70) adapté pour être déployé dans un vaisseau sanguin pour supporter au moins une partie de la paroi interne du vaisseau sanguin.

11. Système médical selon la revendication 10 lorsqu'elle dépend de la revendication 9, dans lequel le stent (60, 70) est doté de marqueurs radio-opaques (63, 73) placés de sorte à être positionnés d'une manière prédéterminée par rapport à la section transversale variable (62, 72) lorsque le ballonnet (61, 71) est correctement aligné à l'intérieur du stent ;
dans lequel au moins deux paires de marqueurs radio-opaques (63, 73) sont de préférence prévues, chaque paire comprenant deux marqueurs agencés de manière diamétralement opposée autour de la circonférence du stent (60, 70).

12. Système selon la revendication 10 ou 11, dans lequel le stent (6, 7, 60, 70) est mobile entre une configuration de pose et une configuration déployée ;
dans lequel le stent est de préférence affaissé dans la configuration de pose ;
dans lequel le stent est de préférence dilaté dans la configuration déployée.

13. Système selon la revendication 12, dans lequel dans la configuration de pose au moins une partie de l'axe longitudinal du stent (6, 7, 60, 70) est sensiblement rectiligne ;
dans lequel dans la configuration déployée, au moins une partie de l'axe longitudinal du stent est de préférence incurvée dans l'espace tridimensionnel, le stent étant de préférence configuré pour exercer une force sur la paroi interne d'un vaisseau sanguin (5), amenant l'axe longitudinal du vaisseau sanguin à s'incurver dans l'espace tridimensionnel.

14. Système selon la revendication 11 ou 12, dans lequel le stent (6, 7, 60, 70) est mobile entre la configuration de pose et une configuration intermédiaire, et entre la configuration intermédiaire et la configuration déployée ;
dans lequel le stent est de préférence au moins partiellement dilaté dans la configuration intermédiaire ;
dans lequel dans la configuration intermédiaire, au moins une partie de l'axe longitudinal du stent est de préférence sensiblement rectiligne ou est incurvée dans l'espace tridimensionnel.

15. Système selon l'une quelconque des revendications 10 à 14, dans lequel dans la configuration dilatée du ballonnet (3, 51, 61, 71), le ballonnet est configuré pour exercer une force via le stent (6, 7, 60, 70) sur la paroi interne d'un vaisseau sanguin (5) pour déformer la paroi interne du vaisseau sanguin de telle sorte que l'axe longitudinal du vaisseau sanguin est amené à s'incurver dans l'espace tridimensionnel.
